# EUROPEAN PATENT APPLICATION

(11) **EP 0 712 932 A2**
(43) Date of publication of application: **22.05.1996**
(21) Application number: 95118149.4
(22) Date of filing: 17.11.1995
(51) Int. Cl.: C12N 15/53, C12N 9/02, C12N 1/19, C12N 15/81, C07K 16/40, C12Q 1/68

(54) **A human flavin-containing monooxygenase**

(30) Priority: 18.11.1994 JP 284902/94
(71) Applicant: SUMITOMO CHEMICAL COMPANY, LIMITED, Osaka-shi, Osaka (JP)
(72) Inventor: Hayashi, Koji, Takarazuka-shi, Hyogo (JP); Matsuki, Yasushi, Nishinomiya-shi, Hyogo (JP); Yabusaki, Yoshiyasu, Kobe-shi, Hyogo (JP)
(74) Representative: VOSSIUS & PARTNER

(57) **Abstract**

A flavin-containing monooxygenase comprising an amino acid sequence set out in SEQ ID NO:7, a microorganism producing said flavin-containing monooxygenase, a flavin-containing monooxygenase gene containing a DNA sequence coding for the amino acid sequence set out in SEQ ID NO:7, a flavin-containing monooxygenase gene containing a DNA sequence set out in SEQ ID NO:7, a plasmid containing said flavin-containing monooxygenase gene, a microorganism containing said plasmid, and a method for producing the enzyme of the present invention wherein said microorganism is cultured to produce the enzyme.

SEQUENCE LISTING

(1) GENERAL INFORMATION:

(i) APPLICANT:
(A) NAME: Sumitomo Chemical Company, Limited
(B) STREET: 5-33, Kitahama 4-chome, Kita-ku
(C) CITY: Osaka-shi, Osaka
(E) COUNTRY: Japan
(F) POSTAL CODE (ZIP): none

(ii) TITLE OF INVENTION: A human flavin-containing monooxygenase

(iii) NUMBER OF SEQUENCES: 8

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: JP 06-284902
(B) FILING DATE: 18-NOV-1994

(2) INFORMATION FOR SEQ ID NO: 1:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 1:

GTCTCGAGGA AGGACTGGAG CCCACCTGCT T 31

(2) INFORMATION FOR SEQ ID NO: 2:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 2:

GCCACACAGA ATGCTTGCTG GGAGCTCATC A 31

(2) INFORMATION FOR SEQ ID NO: 3:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 3:

GTTGCTCGTC ACTCGATTTG GAACCTTCCT C 31

(2) INFORMATION FOR SEQ ID NO: 4:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 4:

AAGAGCTCAG CCCCTGTCTG GGTATTGTCA G 31

(2) INFORMATION FOR SEQ ID NO: 5:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 5:

CTAGAATGGG GAAGAAAGTG GCCATCATTG GAGCTGGTGT GAGTGGCTTG GCCTCCATCA 60

GGAGCTGTC 69

(2) INFORMATION FOR SEQ ID NO: 6:

(i) SEQUENCE CHARACTERISTICS:
(A) LENGTH: 69 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 6:

TTACCCCTTC TTTCACCGGT AGTAACCTCG ACCACACTCA CCGAACCGGA GGTAGTCCTC 60

GACAGAGCT 69

(2) INFORMATION FOR SEQ ID NO: 7:

(i) SEQUENCE CHARACTERISTICS:
(A) LENGTH: 1599 base pairs
(B) TYPE: nucleic acid
(C) STRANDEDNESS: double
(D) TOPOLOGY: linear

(ix) FEATURE:
(A) NAME/KEY: CDS
(B) LOCATION:1..1596

(xi) SEQUENCE DESCRIPTION: SEQ ID NO: 7:

ATG GGG AAG AAA GTG GCC ATC ATT GGA GCT GGT GTG AGT GGC TTG GCC 48
Met Gly Lys Lys Val Ala Ile Ile Gly Ala Gly Val Ser Gly Leu Ala
1               5                   10                  15

TCC ATC AGG AGC TGT CTC GAG GAA GGA CTG GAG CCC ACC TGC TTT GAG 96
Ser Ile Arg Ser Cys Leu Glu Glu Gly Leu Glu Pro Thr Cys Phe Glu
            20                  25                  30

AAG AGC AAT GAC ATT GGG GGC CTG TGG AAA TTT TCA GAC CAT GCA GAG 144
Lys Ser Asn Asp Ile Gly Gly Leu Trp Lys Phe Ser Asp His Ala Glu
        35                  40                  45

GAG GGC AGG GCT AGC ATT TAC AAA TCA GTC TTT TCC AAC TCT TCC AAA 192
Glu Gly Arg Ala Ser Ile Tyr Lys Ser Val Phe Ser Asn Ser Ser Lys
    50                  55                  60

GAG ATG ATG TGT TTC CCA GAC TTC CCA TTT CCC GAT GAC TTC CCC AAC 240
Glu Met Met Cys Phe Pro Asp Phe Pro Phe Pro Asp Asp Phe Pro Asn
65                  70                  75                  80

TTT ATG CAC AAC AGC AAG ATC CAG GAA TAT ATC ATT GCA TTT GCC AAA 288
Phe Met His Asn Ser Lys Ile Gln Glu Tyr Ile Ile Ala Phe Ala Lys
                85                  90                  95

GAA AAG AAC CTC CTG AAG TAC ATA CAA TTT AAG ACA TTT GTA TCC AGT 336
Glu Lys Asn Leu Leu Lys Tyr Ile Gln Phe Lys Thr Phe Val Ser Ser
            100                 105                 110

GTA AAT AAA CAT CCT GAT TTT GCA ACT ACT GGC CAG TGG GAT GTT ACC 384
Val Asn Lys His Pro Asp Phe Ala Thr Thr Gly Gln Trp Asp Val Thr
        115                 120                 125

ACT GAA AGG GAT GGT AAA AAA GAA TCG GCT GTC TTT GAT GCT GTA ATG 432
Thr Glu Arg Asp Gly Lys Lys Glu Ser Ala Val Phe Asp Ala Val Met
    130                 135                 140

GTT TGT TCT GGA CAT CAT GTG TAT CCC AAC CTA CCA AAA GAG TCC TTT 480
Val Cys Ser Gly His His Val Tyr Pro Asn Leu Pro Lys Glu Ser Phe
145                 150                 155                 160

CCA GGA CTA AAC CAC TTT AAA GGC AAA TGC TTC CAC AGC AGG GAC TAT 528
Pro Gly Leu Asn His Phe Lys Gly Lys Cys Phe His Ser Arg Asp Tyr
                165                 170                 175

AAA GAA CCA GGT GTA TTC AAT GGA AAG CGT GTC CTG GTG GTT GGC CTG 576
Lys Glu Pro Gly Val Phe Asn Gly Lys Arg Val Leu Val Val Gly Leu
            180                 185                 190

GGG AAT TCG GGC TGT GAT ATT GCC ACA GAA CTC AGC CGC ACA GCA GAA 624
Gly Asn Ser Gly Cys Asp Ile Ala Thr Glu Leu Ser Arg Thr Ala Glu
        195                 200                 205

CAG GTC ATG ATC AGT TCC AGA AGT GGC TCC TGG GTG ATG AGC CGG GTC 672
Gln Val Met Ile Ser Ser Arg Ser Gly Ser Trp Val Met Ser Arg Val
    210                 215                 220

TGG GAC AAT GGT TAT CCT TGG GAC ATG TTG CTC GTC ACT CGA TTT GGA 720
Trp Asp Asn Gly Tyr Pro Trp Asp Met Leu Leu Val Thr Arg Phe Gly
225                 230                 235                 240

ACC TTC CTC AAG AAC AAT TTA CCG ACA GCC ATC TCT GAC TGG TTG TAC 768
Thr Phe Leu Lys Asn Asn Leu Pro Thr Ala Ile Ser Asp Trp Leu Tyr
                245                 250                 255

GTG AAG CAG ATG AAT GCA AGA TTC AAG CAT GAA AAC TAT GGC TTG ATG 816
Val Lys Gln Met Asn Ala Arg Phe Lys His Glu Asn Tyr Gly Leu Met
            260                 265                 270

CCT TTA AAT GGA GTC CTG AGG AAA GAG CCT GTA TTT AAT GAT GAG CTC 864
Pro Leu Asn Gly Val Leu Arg Lys Glu Pro Val Phe Asn Asp Glu Leu
        275                 280                 285

CCA GCA AGC ATT CTG TGT GGC ATT GTG ACC GTA AAG CCT AAC GTG AAG 912
Pro Ala Ser Ile Leu Cys Gly Ile Val Thr Val Lys Pro Asn Val Lys
    290                 295                 300

GAA TTC ACA GAG ACC TCG GCC ATT TTT GAG GAT GGG ACC ATA TTT GAG 960
Glu Phe Thr Glu Thr Ser Ala Ile Phe Glu Asp Gly Thr Ile Phe Glu
305                 310                 315                 320

GGC ATT GAC TGT GTA ATC TTT GCA ACA GGG TAT AGT TTT GCC TAC CCC 1008
Gly Ile Asp Cys Val Ile Phe Ala Thr Gly Tyr Ser Phe Ala Tyr Pro
                325                 330                 335

TTC CTT GAT GAG TCT ATC ATC AAA AGC AGA AAC AAT GAG ATC ATT TTA 1056
Phe Leu Asp Glu Ser Ile Ile Lys Ser Arg Asn Asn Glu Ile Ile Leu
            340                 345                 350

TTT AAA GGA GTA TTT CCT CCT CTA CTT GAG AAG TCA ACC ATA GCA GTG 1104
Phe Lys Gly Val Phe Pro Pro Leu Leu Glu Lys Ser Thr Ile Ala Val
        355                 360                 365

ATT GGC TTT GTC CAG TCC CTT GGG GCT GCC ATT CCC ACA GTT GAC CTC 1152
Ile Gly Phe Val Gln Ser Leu Gly Ala Ala Ile Pro Thr Val Asp Leu
    370                 375                 380

CAG TCC CGC TGG GCA GCA CAA GTA ATA AAG GGA ACT TGT ACT TTG CCT 1200
Gln Ser Arg Trp Ala Ala Gln Val Ile Lys Gly Thr Cys Thr Leu Pro
385                 390                 395                 400

TCT ATG GAA GAC ATG ATG AAT GAT ATT AAT GAG AAA ATG GAG AAA AAG 1248
Ser Met Glu Asp Met Met Asn Asp Ile Asn Glu Lys Met Glu Lys Lys
                405                 410                 415

CGC AAA TGG TTT GGC AAA AGC GAG ACC ATA CAG ACA GAT TAC ATT GTT 1296
Arg Lys Trp Phe Gly Lys Ser Glu Thr Ile Gln Thr Asp Tyr Ile Val
            420                 425                 430

TAT ATG GAT GAA CTC TCC TCC TTC ATT GGG GCA AAG CCC AAC ATC CCA 1344
Tyr Met Asp Glu Leu Ser Ser Phe Ile Gly Ala Lys Pro Asn Ile Pro
        435                 440                 445

TGG CTG TTT CTC ACA GAT CCC AAA TTG GCC ATG GAA GTT TAT TTT GGC 1392
Trp Leu Phe Leu Thr Asp Pro Lys Leu Ala Met Glu Val Tyr Phe Gly
    450                 455                 460

CCT TGT AGT CCC TAC CAG TTT AGG CTG GTG GGC CCA GGG CAG TGG CCA 1440
Pro Cys Ser Pro Tyr Gln Phe Arg Leu Val Gly Pro Gly Gln Trp Pro
465                 470                 475                 480

GGA GCC AGA AAT GCC ATA CTG ACC CAG TGG GAC CGG TCG TTG AAA CCC 1488
Gly Ala Arg Asn Ala Ile Leu Thr Gln Trp Asp Arg Ser Leu Lys Pro
                485                 490                 495

ATG CAG ACA CGA GTG GTC GGG AGA CTT CAG AAG CCT TGC TTC TTT TTC 1536
Met Gln Thr Arg Val Val Gly Arg Leu Gln Lys Pro Cys Phe Phe Phe
            500                 505                 510

CAT TGG CTG AAG CTC TTT GCA ATT CCT ATT CTG TTA ATC GCT GTT TTC 1584
His Trp Leu Lys Leu Phe Ala Ile Pro Ile Leu Leu Ile Ala Val Phe
        515                 520                 525

CTT GTG TTG ACC TAA 1599
Leu Val Leu Thr
    530

(2) INFORMATION FOR SEQ ID NO: 8:

(i) SEQUENCE CHARACTERISTICS:
(A) LENGTH: 532 amino acids
(B) TYPE: amino acid
(D) TOPOLOGY: linear

(ii) MOLECULE TYPE: protein
(xi) SEQUENCE DESCRIPTION: SEQ ID NO: 8:

Met Gly Lys Lys Val Ala Ile Ile Gly Ala Gly Val Ser Gly Leu Ala
1               5                   10                  15

Ser Ile Arg Ser Cys Leu Glu Glu Gly Leu Glu Pro Thr Cys Phe Glu
            20                  25                  30

Lys Ser Asn Asp Ile Gly Gly Leu Trp Lys Phe Ser Asp His Ala Glu
        35                  40                  45

Glu Gly Arg Ala Ser Ile Tyr Lys Ser Val Phe Ser Asn Ser Ser Lys
    50                  55                  60

Glu Met Met Cys Phe Pro Asp Phe Pro Phe Pro Asp Asp Phe Pro Asn
65                  70                  75                  80

Phe Met His Asn Ser Lys Ile Gln Glu Tyr Ile Ile Ala Phe Ala Lys
                85                  90                  95

Glu Lys Asn Leu Leu Lys Tyr Ile Gln Phe Lys Thr Phe Val Ser Ser
            100                 105                 110

Val Asn Lys His Pro Asp Phe Ala Thr Thr Gly Gln Trp Asp Val Thr
        115                 120                 125

Thr Glu Arg Asp Gly Lys Lys Glu Ser Ala Val Phe Asp Ala Val Met
    130                 135                 140

Val Cys Ser Gly His His Val Tyr Pro Asn Leu Pro Lys Glu Ser Phe
145                 150                 155                 160

Pro Gly Leu Asn His Phe Lys Gly Lys Cys Phe His Ser Arg Asp Tyr
                165                 170                 175

Lys Glu Pro Gly Val Phe Asn Gly Lys Arg Val Leu Val Val Gly Leu
            180                 185                 190

Gly Asn Ser Gly Cys Asp Ile Ala Thr Glu Leu Ser Arg Thr Ala Glu
        195                 200                 205

Gln Val Met Ile Ser Ser Arg Ser Gly Ser Trp Val Met Ser Arg Val
    210                 215                 220

Trp Asp Asn Gly Tyr Pro Trp Asp Met Leu Leu Val Thr Arg Phe Gly
225                 230                 235                 240

Thr Phe Leu Lys Asn Asn Leu Pro Thr Ala Ile Ser Asp Trp Leu Tyr
                245                 250                 255

Val Lys Gln Met Asn Ala Arg Phe Lys His Glu Asn Tyr Gly Leu Met
            260                 265                 270

Pro Leu Asn Gly Val Leu Arg Lys Glu Pro Val Phe Asn Asp Glu Leu
        275                 280                 285

Pro Ala Ser Ile Leu Cys Gly Ile Val Thr Val Lys Pro Asn Val Lys
    290                 295                 300

Glu Phe Thr Glu Thr Ser Ala Ile Phe Glu Asp Gly Thr Ile Phe Glu
305                 310                 315                 320

Gly Ile Asp Cys Val Ile Phe Ala Thr Gly Tyr Ser Phe Ala Tyr Pro
                325                 330                 335

Phe Leu Asp Glu Ser Ile Ile Lys Ser Arg Asn Asn Glu Ile Ile Leu
            340                 345                 350

Phe Lys Gly Val Phe Pro Pro Leu Leu Glu Lys Ser Thr Ile Ala Val
        355                 360                 365

Ile Gly Phe Val Gln Ser Leu Gly Ala Ala Ile Pro Thr Val Asp Leu
    370                 375                 380

Gln Ser Arg Trp Ala Ala Gln Val Ile Lys Gly Thr Cys Thr Leu Pro
385                 390                 395                 400

Ser Met Glu Asp Met Met Asn Asp Ile Asn Glu Lys Met Glu Lys Lys
                405                 410                 415

Arg Lys Trp Phe Gly Lys Ser Glu Thr Ile Gln Thr Asp Tyr Ile Val
            420                 425                 430

Tyr Met Asp Glu Leu Ser Ser Phe Ile Gly Ala Lys Pro Asn Ile Pro
        435                 440                 445

Trp Leu Phe Leu Thr Asp Pro Lys Leu Ala Met Glu Val Tyr Phe Gly
    450                 455                 460

Pro Cys Ser Pro Tyr Gln Phe Arg Leu Val Gly Pro Gly Gln Trp Pro
465                 470                 475                 480

Gly Ala Arg Asn Ala Ile Leu Thr Gln Trp Asp Arg Ser Leu Lys Pro
                485                 490                 495

Met Gln Thr Arg Val Val Gly Arg Leu Gln Lys Pro Cys Phe Phe Phe
            500                 505                 510

His Trp Leu Lys Leu Phe Ala Ile Pro Ile Leu Leu Ile Ala Val Phe
        515                 520                 525

Leu Val Leu Thr
    530

## Description

The present invention relates to a novel human flavin-containing monooxygenase (FMO) gene.

Flavin-containing monooxygenases of rat, pig and rabbit have been known as microsomal xenobiotic-metabolizing enzymes which oxidize various xenobiotics including drugs, agricultural chemicals and environmental pollutants incorporated into bodies, and it has also been known that they have a plurality of isozymes. Therefore, precise characterization and specific function of each enzyme molecule have not been thoroughly understood. Particularly, with regard to human flavin-containing monooxygenases, only 3 human flavin-containing monooxygenases have been elucidated by cloning of cDNA (Dolphin et al., J. Biol. Chem., 266, 12379-12385, (1991), Dolphin et al., Biochem. J., 287, 261-267 (1992), Lomri et al. Proc. Natl. Acad. Sci. 89,1685-1689 (1992)). Therefore, many aspects of human flavin-containing monooxygenases, such as population distribution of the isozymes in human and their function to metabolize or detoxify xenobiotics, remain unknown.

To solve this problem, the present inventors have found a novel human flavin-containing monooxygenase and produced in a large quantity a functional human flavin-containing monooxygenase by a DNA amplification technique using particular oligonucleotides as primers and a genetic engineering method.

Thus, the present invention provides nucleotide sequences encoding a human flavin-containing monooxygenase, wherein these nucleotide sequences are RNA and DNA sequences, such as a flavin-containing monooxygenase gene containing a DNA sequence coding for the amino acid sequence set out in SEQ ID NO:7,
a flavin-containing monooxygenase gene containing a DNA sequence as set out in SEQ ID NO:7 (hereinafter, referred to as the gene of the invention),
a vector containing said flavin-containing monooxygenase gene (hereinafter, referred to as the vector of the invention), a host cell containing said vector, and a method for producing the enzyme of the present invention, which comprises culturing said host cell and recovering the enzyme it produces.

The host cell of the invention includes, but is not limited to, microorganisms such as yeast. The vector of the invention includes, but is not limited to, plasmid and phage vectors, such as a plasmid suitable for expression in yeast. The present invention also provides nucleotide sequences that hybridize to the above-mentioned nucleotide sequences and that encode an enzyme having the xenobiotic-oxidizing activity of human flavin-containing monooxygenase. In this context, the term "hybridization" refers to conventional hybridization conditions and preferably to stringent hybridization conditions. The present invention also provides an enzyme encoded by any one of the above-mentioned nucleotide sequences or produced by the above method.

The present invention further provides an antibody, preferably monoclonal, which specifically binds to an epitope of the enzyme of the invention. The present invention further provides an oligonucleotide probe which specifically binds the nucleotide sequence of the invention.

The present invention additionally provides a cell-free extract, preferably a microsomal fraction, prepared from the host cell of the invention. The present invention further provides a method for metabolizing a sample compound comprising preparing a mixture of the sample compound and the host cell or cell-free extract of the invention, incubating the mixture and, optionally analyzing the metabolite so produced.

The above and other objects, features, and advantages of the present invention will be better understood from the following detailed descriptions taken in conjunction with the accompanying drawing, which is given by way of illustration only and is not limitative of the present invention, in which:

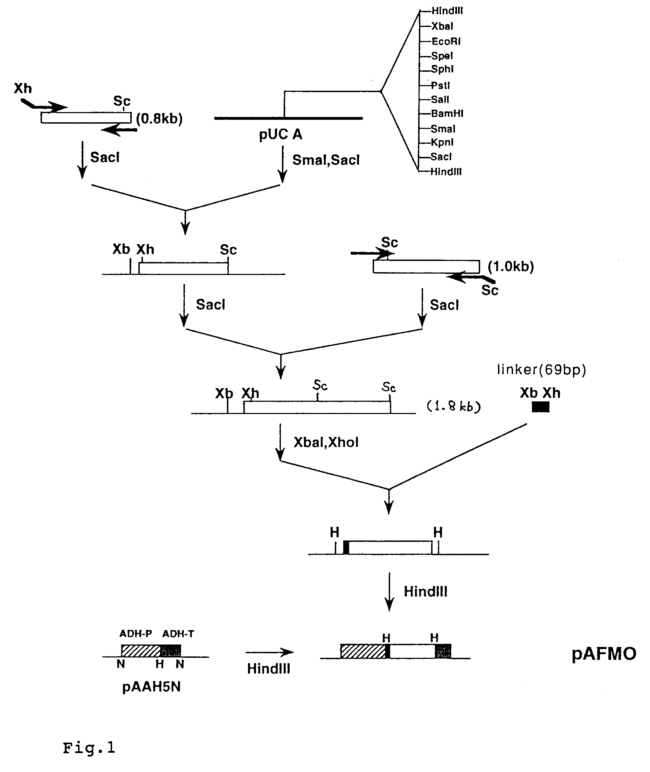

Figure 1 shows a construction method of a yeast expression plasmid (pAFMO) for a human flavin-containing monooxygenase.

The present invention provides a novel human flavin-containing monooxygenase, a host cell which produces said enzyme, a nucleotide sequence comprising said enzyme gene and nucleotide sequences hybridizing therewith, and a vector containing said nucleotide sequence. The expressed enzyme of the present invention is useful to evaluate human xenobiotic metabolism in vitro.

The invention will be described in detail as follows:
The enzyme of the present invention is novel, and differs from any of the aforementioned three known human flavin-containing monooxygenases. Its amino acid sequence or the DNA sequence coding for said sequence has about 50 to 95% homology with those of the amino acid sequences or DNA sequences of the aforementioned known enzymes, respectively.

The gene of the present invention can be prepared by a conventional genetic engineering method, for example, which comprises cloning subject gene prepared from a cDNA library. The cDNA library can be obtained by the steps of;
Preparing an mRNA fraction of said gene, producing cDNA using reverse transcriptase, and inserting said cDNA into a vector, preferably a phage vector or a plasmid vector. Alternatively, a commercially available cDNA library derived from human liver may be screened using either (i) a DNA fragment which exhibits homology with the gene or (ii) an antibody which recognizes the protein produced by the gene. Furthermore, the gene can be prepared by cloning the subject gene from the cDNA library described above by PCR using a specific oligonucleotide as a primer.

A specific oligonucleotide to be used as a primer in PCR includes, for example, DNA fragments as set forth in SEQ ID NO:1 to 4. When said primers are used, about 1.6 Kb fragment corresponding to the protein coding region of human flavin-containing monooxygenase gene excluding 60 bp of N-terminal can be amplified separately as two fragments of about 0.8 Kb and about 1.0 Kb. The resulting two fragments, about 0.8 Kb and about 1.0 Kb, and the fragment corresponding to the N-terminal of about 60 bp (linker) are ligated by a conventional genetic engineering method, thus the preparation of the gene of the present invention is accomplished.

The vector of the present invention is usually constructed by inserting the gene of the present invention into an expression vector. The expression vector usually must contain genetic information that can be replicated in host cells, propagate independently, be readily isolated and purified from the host cells and further contain a detectable marker. Such a vector can be constructed by a conventional genetic engineering method.

In a yeast expression system, for example, a yeast expression plasmid can be prepared by inserting the gene of the present invention obtained by cloning into an expression vector containing a promoter and terminator which are functional in yeast.

A functional promoter in yeast to be used in the invention includes, e.g., the promoters of a yeast alcohol dehydrogenase gene (hereinafter, referred to as ADH promoter), a glyceraldehyde-3-phosphate dehydrogenase gene (hereinafter, referred to as GAPDH promoter), and a phosphoglycerate kinase gene (hereinafter, referred to as PGK promoter).

The ADH promoter can be prepared, for example, from the yeast expression vector pAAH5 containing yeast ADH1 promoter and terminator (available from Washington Research Foundation, Ammerer et al. Methods in Enzymology, 101, p192-201) by a conventional genetic engineering method. Since the yeast ADH1 promoter is disclosed by U.S. Pat. No. 299,733 (1981/9/31) of Washington Research Foundation, industrial or commercial use of the the promoter requires approval of the patentee. Said yeast expression plasmid containing the aforementioned promoter and terminator which exert their functions in yeast, and human flavin-containing monooxygenase gene can be readily constructed by a conventional genetic engineering method. For example, the plasmid can be constructed by inserting the human flavin-containing monooxygenase gene into the HindIII sites of the yeast expression vector pAAH5N containing the ADH promoter and ADH terminator disclosed in JP-A No.21180/1990.

The resulting vector of the present invention is introduced into a host cell by a conventional method to produce the transformant host cell. Then, said host cell is cultured to produce the enzyme of the present invention selectively and in a large quantity.

For example, the vector of the present invention is introduced into yeast such as Saccharomyces cerevisiae according to a method such as an alkali metal (LiCl) method. The yeast into which said vector is introduced is then cultured to produce the enzyme of the present invention by a conventional method.

The expressed enzyme is localized in the microsomal membrane and exerts its monooxygenase activity. Therefore, the expressed enzyme can, for example, be used to investigate metabolism of a xenobiotic in vitro and is preferably used in a form of intact yeast cells or cell-free extracts. A cell-free extract includes, for example, a microsomal fraction. The preparation of the cell-free extracts or the microsomal fraction may be performed according to a conventional method described, for example, in DNA, vol. 4. No.3, page 203 - page 210 (1985).

The yeast cells or the cell-free extracts thus obtained may be used to analyze a metablic pathway of a sample compound by reacting the sample compound with the yeast cells or the cell-free extracts. The reaction can be performed by adding the sample compound to a culture of the the yeast cells or a solution of cell-free extracts such as culture medium or buffer containing the yeast cells or the cell-free extracts, and by incubating the reaction mixture, for example, at a temperature of about 10 to 40°C, for about 0.1 to 48 hours. The amount of the yeast cells or said cell-free extracts and the amount of the sample compound to be added to the reaction mixture may be varied according to various conditions such as the reaction temperature, reaction time and the type of the sample compound. For example, the amount of the yeast cells or said cell-free extracts is preferably between about 10⁷ and about 10⁸ cells or about 5 to 200 µl of the microsomal fraction (per 1 ml of the solution) and the amount of the sample compound to be added to the reaction mixture is preferably between about 0.01 and 1 µmole per 1 ml of the solution. These amounts can be optionally increased or decreased irrespective of a limitation of the above-described ranges.

After completion of the reaction, analysis of metabolites in the reaction solution can be conducted according to a conventional analytical method described in Guideline of Instrumental Analysis (2) (New edition first published 1985), KAGAKU-DOJIN Publishing Company edited by Jiro Shiokawa et al, or Spectrometric Identification of Organic compounds (Fourth edition third published 1984), TOKYO KAGAKU DOJIN Co., Ltd. Publishers edited by R. M. Silver et al.. On the basis of the analytical data above, it can be judged as to whether the sample compound is either detoxified to a metabolite or activated to a carcinogen by the present enzyme.

The invention will be further illustrated with reference to the following examples; however, these examples are not to be construed to limit the scope of the invention.

### EXAMPLE 1: METHOD FOR OBTAINING THE GENE

Using the primers set out in SEQ ID NO:1 to 4, about 1.6 Kb fragment corresponding to the protein coding region of human flavin-containing monooxygenase gene without 60 bp of N-terminals was amplified separately as two fragments: about 0.8 Kb and about 1.0 Kb, according to PCR. One of the amplified fragments of about 0.8 Kb was cleaved with SacI and subcloned into the SmaI-SacI-site of the pUC A vector, which was prepared by modifying the EcoRI site of pUC19 (TAKARA SHUZO) into the HindIII site, and by converting the cloning sites between the HindIII sites into the following cloning sites: 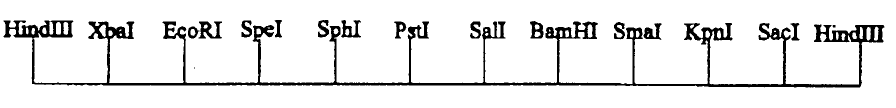
HindIII XbaI EcoRI SpeI SphI PstI SalI BamHI SmaI KpnI SacI HindIII
The obtained subclone was treated with SacI, and ligated to the fragment of about 1.0 Kb which was pretreated with SacI, and then, the resulting plasmid was further treated with XbaI and XhoI into which the linkers set out in SEQ ID NO:5 and 6 were inserted (see Figure 1). The gene corresponding to the protein coding region of human flavin-containing monooxygenase was sequenced using a fluorescence DNA sequencer (model 373A, Applied Biosystems) which is based on the dideoxy method. The result is shown in SEQ ID NO:7, accompanied by the deduced amino acid sequence.

### EXAMPLE 2: CONSTRUCTION OF THE PLASMID

The gene corresponding to the protein coding region of human flavin-containing monooxygenase was prepared by cleaving the obtained plasmid with HindIII, and inserted into pAAH5N, whereby the yeast expression plasmid pAFMO was constructed, which allowed the gene of the invention to express in yeast (see Figure 1).

### EXAMPLE 3: PRODUCTION OF THE MICROORGANISM

Saccharomyces cerevisiae AH22 was inoculated in 1 ml of YPD medium (1%(w/v) yeast extract, 2%(w/v) polypeptone, and 2%(w/v) glucose), cultivated at 30°C for 18 hours, and then collected by centrifugation (10,000 x g, 2 minutes, room temperature). The obtained cells were suspended in 1 ml of 0.2 M LiCl solution, then centrifuged again, and to the resulting pellet were added 20 µl of 1 M LiCl, 30 µl of 70%(w/v) polyethyleneglycol 4000 solution and 10 µl of the solution containing about 1.0 µg of the plasmid of the present invention (pAFMO) constructed in Example 2. The resulting solution was thoroughly mixed, then incubated at 30°C for 1 hour, further added to 140 µl of distilled water, and stirred. The solution was spread on the SD synthetic plate (2.0%(w/v) glucose, 0.67%(w/v) yeast nitrogen base without amino acid, 20 g/ml Histidine, 2.0%(w/v) agar), incubated at 30°C for 3 days, and the tansformant containing the plasmid of the present invention (pAFMO) was obtained.

### EXAMPLE 4: PREPARATION OF THE MICROSOMAL FRACTION OF YEAST

The microorganism of the present invention (a transformed yeast produced in Example 3) was collected from 3.8 liter of the liquid medium in which the microorganism was cultured up to about 1.0 x 10⁸ cells/ml. The yeast cells were suspended in 400 ml of buffer A (10 mM Tris-HCl (pH7.5), 2 M sorbitol, 0.1 mM DTT, 0.2 mM EDTA), and to the resulting solution was added 160 mg of Zymolyase 100T, and incubated at 30 °C for 60 minutes. After suspending spheroplasts obtained by centrifugation (5,000 x g, 10 minutes, 4°C) in 100 ml of buffer A, the resulting solution was subjected to centrifugation (5,000 x g, 10 minutes, 4°C). After washing the spheroplasts by subjecting the solution to centrifugation again under the same conditions, the spheroplasts were suspended in 200 ml of the buffer (10 mM Tris-HCl (pH7.5), 0.65 M sorbitol, 0.1 mM DTT), and disrupted by ultrasonication (50 W, 5 minutes, 0°C). The supernatant obtained by centrifugation (9,000 x g, 20 minutes, 4°C) is referred to as yeast S-9 Mix fraction hereinafter. This fraction was further centrifuged (125,000 x g, 70 minutes, 4°C) to collect pellets, which were then suspended in 10 ml of 0.1 M phosphate buffer (pH7.4) to obtain a microsomal fraction.

### EXAMPLE 5: ASSAY OF THIOUREA S-OXYGENASE ACTIVITY IN THE MICROSOMAL FRACTION OF YEAST WHICH EXPRESSES THE ENZYME

The reaction was initiated by adding 200 µl of the microsomal fraction prepared in Example 4 and 25 µl of 120 mM thiourea to 2.5 ml of an assay solution (0.1 M potassium phosphate buffer, pH7.5, 0.2 mM NADPH, 160 µM thiocholine, 100 units catalase, 2 mM benzylimidazole, 0.4 mM EDTA) previously warmed at 37°C. 400 µl of the mixture was added to 40 µl of 3 M TCA every 3 minutes, the resulting mixture was left standing on ice, and then centrifuged to obtain 350 µl of a supernatant. To the supernatant were added 1 ml of 1 M potassium phosphate buffer (pH7.5), 0.6 ml of water and 50 µl of 10 mM dithiobisnitrobenzoic acid for coloration, and decrease of thiocholine was measured with an absorption at 412 nm. Thiourea S-oxygenase activity was found in the yeast microsomal fraction expressing the present enzyme.

## Claims

1. A nucleotide sequence encoding an enzyme having the xenobiotic oxidizing activity of human flavin-containing monooxygenase comprising:
(a) a nucleotide sequence as set out in SEQ ID NO:7;
(b) a nucleotide sequence coding for the amino acid sequence set out in SEQ ID NO:7; or
(c) a nucleotide sequence which hybridizes with (a) or (b).

2. The nucleotide sequence of claim 1 which is DNA.

3. The nucleotide sequence of claim 1 which is RNA.

4. A vector comprising the nucleotide sequence of any one of claims 1 to 3.

5. The vector of claim 4 which is a plasmid or a phage vector.

6. The vector of claim 4 or 5 wherein said nucleotide sequence is operably linked to an expression control sequence.

7. A host cell containing the vector of any one of claims 4 to 6.

8. The host cell of claim 7 which is a yeast cell.

9. A method for producing an enzyme having the xenobiotic oxidizing activity of human flavin-containing monooxygenase, comprising:
(a) culturing the host cell of claim 7 or 8; and
(b) recovering the enzyme so produced.

10. An enzyme having the xenobiotic oxidizing activity of human flavin-containing monooxygenase and encoded by the nucleotide sequence of any one of claims 1 to 3 or produced by the method of claim 9.

11. An antibody which specifically binds to an epitope of the enzyme of claim 10.

12. The antibody of claim 11 which is a monoclonal antibody.

13. An oligonucleotide probe which specifically binds the nucleotide sequence of any one of claims 1 to 3.

14. A cell-free extract prepared from the host cell of claim 7 or 8.

15. The cell free extract of claim 14 which is a microsomal fraction.

16. A method for metabolizing a sample compound, comprising;
(a) preparing a mixture of the sample compound and the host cell of claim 7 or 8 or the cell-free extract of claim 14 or 15;
(b) incubating the mixture of (a); and, optionally,
(c) analyzing the metabolite so produced.
